(19) Europäisches Patentamt
European Patent Office
Office européen des brevets

(11) Veröffentlichungsnummer : **0 395 982 B1**

(12) # EUROPÄISCHE PATENTSCHRIFT

(45) Veröffentlichungstag der Patentschrift : **20.01.93 Patentblatt 93/03**

(51) Int. Cl.[5] : **C07C 255/07**, C07C 255/34, C07C 255/37, A61K 7/46, C11B 9/00

(21) Anmeldenummer : **90107705.7**

(22) Anmeldetag : **24.04.90**

(54) **Beta,gamma-Ungesättigte Nitrile, deren Herstellung und Verwendung als Riechstoffe.**

(30) Priorität : **29.04.89 DE 3914391**

(43) Veröffentlichungstag der Anmeldung : **07.11.90 Patentblatt 90/45**

(45) Bekanntmachung des Hinweises auf die Patenterteilung : **20.01.93 Patentblatt 93/03**

(84) Benannte Vertragsstaaten : **CH DE ES FR GB LI NL**

(56) Entgegenhaltungen :
**EP-A- 0 074 253**
**DE-A- 2 333 265**
**DE-A- 2 635 545**
**DE-B- 2 256 483**
**US-A- 3 531 510**

(73) Patentinhaber : **BASF Aktiengesellschaft**
**Carl-Bosch-Strasse 38**
**W-6700 Ludwigshafen (DE)**

(72) Erfinder : **Lauterbach, Gerald, Dr.**
**Arminstrasse 38a**
**W-6140 Bensheim (DE)**
Erfinder : **Becker, Rainer, Dr.**
**Im Haseneck 22**
**W-6702 Bad Duerkheim (DE)**
Erfinder : **Jansen, Klaas**
**Tannenstrasse 30**
**W-6700 Ludwigshafen (DE)**

EP 0 395 982 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ungesättigte Nitrile der allgemeinen Formel I

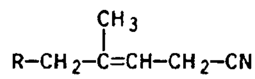 (I),

in der

R für einen verzweigten oder unverzweigten Alkylrest mit 4 bis 10 C-Atomen oder einen Phenylring steht, der durch eine Alkylgruppe mit 1 bis 4 C-Atomen, eine oder mehrere Alkoxygruppen mit 1 bis 3 C-Atomen, vorzugsweise eine Methoxygruppe oder eine Alkylendioxygruppe, vorzugsweise eine Methylendioxygruppe substituiert ist,

sowie deren Herstellung und deren Verwendung zur Verleihung, Verbesserung oder Modifizierung der Dufteigenschaften von Parfümen oder zu parfümierenden Produkten.

Aus DE-A-2635545, DE-B-2256483, US-A-3531510 sind Riechstoffe auf der Basis von strukturell verwandten ungesättigten Nitrilen bekannt.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmitteln, Kosmetika, Leimen hat die Riechstoffindustrie ständig Bedarf an neuen Riechstoffen, die allein oder in Form von Kompositionen wertvolle Parfüms bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es war daher die Aufgabe der Erfindung, neue interessante Riechstoffe zu entwickeln, die auf möglichst einfache weise aus gut zugänglichen und daher billigen Ausgangsstoffen hergestellt werden können und die außerdem eine gute Stabilität in den verschiedensten Medien aufweisen.

Die erfindungsgemäßen ungesättigten Nitrile der Formel I erfüllen die genannten Anforderungen in überraschend hohem Maße.

Von besonderer Bedeutung sind ungesättigte Nitrile der allgemeinen Formel I, in der R für

$$-(CH_2)_n-CH_3$$

steht, worin n eine ganze Zahl von 5 bis 8 bedeutet, sowie Nitrile der allgemeinen Formel I, in der R einen aromatischen Rest der Formel bedeutet,

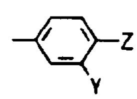

worin Y = H, wenn Z = $-CH_3$;

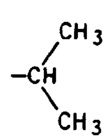 ; 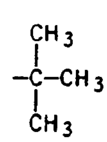 oder $-O-CH_3$

bedeutet oder Y und Z für $-O-CH_3$ stehen oder aber Y und Z zusammen für den Rest

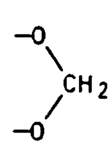

stehen.

Genannt seien insbesondere 4,7,9,9-Tetramethyl-dec-3-en-nitril, 4-Methyl-tridec-3-en-nitril, 4-Methyl-dodec-3-en-nitril, 5-(4-Methyl-phenyl)-4-methyl-pent-3-en-nitril sowie 5-(4-tert.Butyl-phenyl)-4-methyl-pent-3-en-nitril.

Die Nitrile der Formel I können auf einfache weise durch Umsetzen der in der Regel gut und preiswert verfügbaren Aldehyde der allgemeinen Formel II

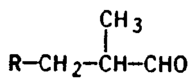 (II),

in der R die oben angegebene Bedeutung hat, in einer Knoevenagel-Reaktion mit Cyanessigsäure hergestellt werden.

Hierzu erhitzt man den Aldehyd der Formel II in einem inerten, mit Wasser nicht mischbaren Lösungsmittel, wie Benzol, Toluol oder Xylol oder in einem basischen Lösungsmittel, wie Dimethylformamid oder Piperidin (Knoevenagel-Doebner-Reaktion) oder aber in Gemischen solcher Lösungsmittel und ggf. in Gegenwart von Ammoniumacetat oder Eisessig unter Wasserabscheidung bis keine Wasserabscheidung mehr beobachtet werden kann. Die Aufarbeitung des Reaktionsgemisches erfolgt bei Verwendung inerter Lösungsmittel im allgemeinen destillativ, bei Mitverwendung von basischen Lösungsmitteln vielfach durch Schütten des Reaktionsansatzes auf Eis und anschließende Extraktion. Nähere Einzelheiten über die Durchführung von solchen Knoevenagel-Reaktionen finden sich beispielsweise im "Organikum", VEB Deutscher Verlag der Wissenschaften, Berlin, 1986, Seiten 458-461.

In den Fällen, in denen die Allylhalogenide der allgemeinen Formel III

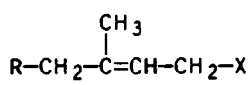 (III),

in der R die oben angegebene Bedeutung hat und X für Cl, Br oder J steht, gut zugänglich sind, ist es von Vorteil die Nitrile der Formel I dadurch herzustellen, daß man die Allylhalogenide der allgemeinen Formel III mit Cyanidionen umsetzt. (Vgl. "Organikum", VEB Deutscher Verlag der Wissenschaften, Berlin, 1986, Seiten 210 bis 213). In manchen Fällen ist es von Vorteil die Umsetzung der Allylhalogenide der Formel III mit Cyanidionen unter Phasentransferkatalyse durchzuführen.

Bezüglich näherer Einzelheiten über Umsetzungen unter Phasentransferkatalyse verweisen wir beispielsweise auf Dehmlow, Angewandte Chemie, 86 (1974), Seiten 187-197.

Gegenstand der Erfindung ist neben den ß,$\gamma$-ungesättigten Nitrilen der allgemeinen Formel I und deren Herstellung durch eine Knoevenagel-Reaktion der Aldehyde der allgemeinen Formel II mit Cyanessigsäure bzw. eine Umsetzung der Allylhalogenide der allgemeinen Formel III mit Cyanidionen auch die Verwendung der Nitrile der Formel I zur Verleihung, Verbesserung oder Modifizierung der Dufteigenschaften von Parfüms oder zu parfümierenden Produkten.

Da die Geruchsrichtungen der Nitrile stark von dem Rest R abhängig sind, kann durch eine Variation dieses Restes eine breite Palette von neuen Riechstoffen nach diesen Verfahren auf einfachem und kostengünstigem weg hergestellt werden, insbesondere da die entsprechenden Aldehyde der Formel II in der Regel gut und preiswert verfügbar sind. Neben ihren interessanten Geruchseigenschaften und ihrer einfachen Synthese besitzen die neuen Nitrile außerdem eine überraschend hohe Stabilität in neutralen, wie auch schwach sauren und basischen Medien, so daß für sie ein außergewöhnlich großer Anwendungsbereich in Frage kommt.

In den nachfolgenden Beispielen wird die Herstellung zahlreicher ungesättigter Nitrile der Formel I beschrieben. Neben den physikalischen Daten werden hier auch die jeweiligen Duftnoten der neuen Verbindungen angegeben.

Beispiele 1 bis 8

Jeweils 2 mol des aus Tabelle 1 ersichtlichen Aldehyds wurden mit 180 g (2,2 mol) Cyanessigsäure, 600 ml Toluol und 23,4 g Ammoniumacetat unter Rühren 3 Stunden (h) unter ständigem Abscheiden von Wasser erhitzt. Anschließend kühlte man das Reaktionsgemisch ab und wusch es mit 500 ml Wasser. Nach dem Abtrennen der wäßrigen Phase wurde die organische Phase unter stark vermindertem Druck destilliert. In der

folgenden Tabelle 1 sind die Namen, Ausbeuten, Siedepunkte und Duftnoten der erhaltenen ungesättigten Nitrile angegeben.

Tabelle 1

| Beispiel | Aldehyd | erhaltenes Nitril | Ausbeute [g (% d.Th.)] | Kp [°C/mbar] | Duftnote |
|---|---|---|---|---|---|
| 1 | 2-Methyl-undecanal | 4-Methyl-tridec-3-en-nitril | 320 (77 %) | 127-130/0,5 | aldehydig, frisch (intensiv) |
| 2 | 2-Methyl-decanal | 4-Methyl-dodec-3-en-nitril | 290 (75 %) | 107-113/0,5 | frisch, citrus, blumig (intensiv) |
| 3 | 3-(4-Methyl-phenyl)-2-methyl-propanal | 5-(4-Methyl-phenyl)-4-methyl-pent-3-en-nitril | 270 (73 %) | 129-136/0,5 | jasmin, citrus, blumig |
| 4 | 3-(4-Isopropyl-phenyl)-2-methyl-propanal | 5-(4-Isopropyl-phenyl)-4-methyl-pent-3-en-nitril | 260 (61 %) | 129-132/0,4 | aldehydig, wachsartig |
| 5 | 3-(4-tert.-Butyl-phenyl)-2-methyl-propanal | 5-(4-tert.-Butyl-phenyl)-4-methyl-pent-3-en-nitril | 350 (77 %) | 139-142/0,3 | blumig |
| 6 | 3-(4-Methoxy-phenyl)-2-methyl-propanal | 5-(4-Methoxy-phenyl)-4-methyl-pent-3-en-nitril | 250 (61 %) | 136-140/0,5 | süß, krautig |
| 7 | 3-(3,4-Dioxymethylen-phenyl)-2-methyl-propanal | 5-(3,4-Dioxymethylen-phenyl)-4-methyl-pent-3-en-nitril | 268 (58 %) | 150-155/0,5 | blumig, grün |
| 8 | 3-(3,4-Dimethoxy-phenyl)-2-methyl-propanal | 5-(3,4-Dimethoxy-phenyl)-4-methyl-pent-3-en-nitril | 268 (58 %) | 150-155/0,35 | süß |

Beispiel 9

Synthese von 4, 7, 9, 9-Tetramethyl-dec-3-en-nitril

438 g (2,38 mol) 2, 5, 7, 7-Tetramethyl-octanal, 202 g (2,38 mol) Cyanessigsäure, 350 ml Toluol, 350 ml Dimethylformamid und 28,5 g Ammoniumacetat wurden unter Rühren 4 h am Wasserabscheider erhitzt. Anschließend wurde das Toluol abdestilliert und der verbleibende Rückstand 2 h auf 180°C erwärmt. Der abgekühlte Rückstand wurde auf Eiswasser gegossen und mit Diethylether extrahiert. Die organische Phase wurde nacheinander mit $NaHCO_3$-Lösung und Wasser gewaschen und anschließend destilliert. Man erhielt 450 g 4, 7, 9, 9-Tetramethyl-dec-3-en-nitril mit einem Siedepunkt von 83 bis 88°C/0,3 mbar (Ausbeute: 91 % d. Th.) Duftnote: wachsartig, blumig, frisch.

Beispiel 10 (Anwendungsbeispiel)

Die großen Einsatzmöglichkeiten der neuen Nitrile sollen beispielhaft anhand der Wirkung des 4-Methyl-tridec-3-en-nitrils (Beispiel 1) in einer Komposition gezeigt werden. Wegen der großen Geruchsintensität und der ausgezeichneten Haftfähigkeit dieser Verbindung bewirkten schon geringe Zusätze in Kompositionen überzeugende Effekte.

| | |
|---|---|
| Dihydromyrcenol | 0,30 g |
| Lysmeral® (BASF) | 1,40 g |
| p-tert-Butyl-cyclohexylacetat | 1,80 g |
| Phenylethylmethylether | 0,30 g |
| Jasmorange® (BASF) | 0,50 g |
| Galaxolid® 50 (IFF) | 0,70 g |
| Cedrylacetat | 0,40 g |
| Styrollylacetat | 0,10 g |
| Lavandin Abrialis | 0,50 g |
| Tetrahydrolinalool | 1,30 g |
| Terpinylacetat | 1,10 g |
| Alkohol $C_9$ | 0,30 g |
| Cyclohexylethanol | 0,10 g |
| Dihydrorosenoxid | 0,30 g |
| Terpineol | 0,20 g |
| Phenylessigsäure 10 % in DPG* | 0,10 g |
| Dimethylheptanol | 0,10 g |
| Citronellol | 0,30 g |
| 4-Methyl-tridec-3-en-nitril | 0,20 g |
| | 10,00 g |

* DPG = Dipropylenglykol

Das erfindungsgemäße 4-Methyl-tridec-3-en-nitril verlieh der Komposition eine sehr interessante, frische, blumige Note mit ozonartiger Nebennote.

Beispiel 11 (Stabilitätsvergleich)

Zum Nachweis der vielseitigen Anwendbarkeit und der besseren Stabilität gegenüber einem gängigen Riechstoff mit sehr ähnlicher Duftnote wurde ein in der Riechstoffbranche üblicher Stabilitätsvergleich des erfindungsgemäßen 4-Methyl-tridec-3-en-nitrils mit dem handelsüblichen Riechstoff Methylnonylacetaldehyd ($C_{12}$MNA) durchgeführt. Hierzu wurden Proben der Riechstoffe in verschiedene übliche Handelsprodukte und

in wäßrige Lösungen mit verschiedenen pH-Werten eingearbeitet und bei Raumtemperatur und bei 35°C über einen Zeitraum von 4 Monaten die Duftnoten der Testprodukte überprüft. Wie der folgenden Tabelle zu entnehmen ist, ist die Stabilität der erfindungsgemäßen Verbindung der des bekannten Riechstoffs in vielen Anwendungsbereichen überlegen.

In der folgenden Tabelle bedeutet:

+ = stabil
± = bedingt stabil
- = nicht stabil

| Testmedium | 4-Methyl-tridec--3-en-nitril | $C_{12}$-MNA |
|---|---|---|
| Seife | + | + |
| Waschpulver (Perborat) | + | + |
| Waschpulver (TAED) | ± | - |
| Weichspüler (Stabilität) | + | + |
| Weichspüler (Haftung auf Textilien) | + | ± |
| Shampoo | + | ± |
| Bleichlauge | + | ± |
| Kaltwellpräparat | + | ± |
| Antiperspirant | + | + |

| Testmedium | 4-Methyl-tridec--3-en-nitril | $C_{12}$-MNA |
|---|---|---|
| pH 1 | - | - |
| pH 2 | - | ± |
| pH 3 | ± | ± |
| pH 4 | + | + |
| pH 9 | + | + |
| pH 10 | + | ± |
| pH 11 | + | ± |
| pH 12 | ± | - |

**Patentansprüche**

**Patentansprüche für folgende Vertragsstaaten : CH, DE, FR, GB, LI, NL**

1. Ungesättigte Nitrile der allgemeinen Formel I

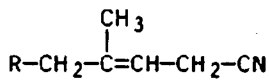

(I),

in der

R für einen verzweigten oder unverzweigten Alkylrest mit 4 bis 10 C-Atomen oder einen Phenylring steht, der durch eine Alkylgruppe mit 1 bis 4 C-Atomen, eine oder mehrere Alkoxygruppen mit 1 bis 3 C-Atomen oder eine Alkylendioxygruppe substituiert ist.

2. Ungesättigte Nitrile der allgemeinen Formel I gemäß Anspruch 1, in der R für

$$-(CH_2)_n-CH_3$$

steht, worin n eine ganze Zahl von 5 bis 8 bedeutet.

3. Ungesättigte Nitrile der allgemeinen Formel I gemäß Anspruch 1, in der R einen aromatischen Rest der Struktur

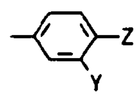

bedeutet, worin Y = H, wenn Z für $-CH_3$;

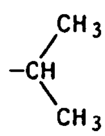 ; 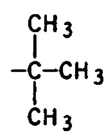 oder $-O-CH_3$

steht oder Y und Z für $-O-CH_3$ stehen oder aber Y und Z zusammen für den Rest

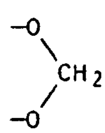

stehen.

4. 4, 7, 9, 9-Tetramethyl-dec-3-en-nitril.

5. 4-Methyl-tridec-3-en-nitril.

6. 4-Methyl-dodec-3-en-nitril.

7. 5-(4-Methyl-phenyl)-4-methyl-pent-3-en-nitril.

8. 5-(4-tert.-Butyl-phenyl)-4-methyl-pent-3-en-nitril.

9. Verfahren zur Herstellung der ungesättigten Nitrile der allgemeinen Formel I gemäß Anspruch 1, dadurch

gekennzeichnet, daß man Aldehyde der allgemeinen Formel II

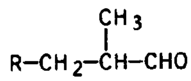

(II),

in der R die in Anspruch 1 angegebene Bedeutung hat, in einer Knoevenagel-Reaktion mit Cyanessigsäure umsetzt.

**10.** Verfahren zur Herstellung der ungesättigten Nitrile der allgemeinen Formel I gemäß Anspruch 1, dadurch gekennzeichnet, daß man Allylhalogenide der allgemeinen Formel III

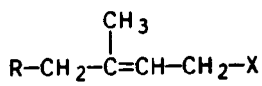

(III),

in der R die in Anspruch 1 angegebene Bedeutung hat und X für Cl, Br oder J steht, ggf. unter Phasentransferkatalyse mit Cyanidionen umsetzt.

**11.** Verwendung der ungesättigten Nitrile der allgemeinen Formel I gemäß Anspruch 1 zur Verleihung, Verbesserung oder Modifizierung der Dufteigenschaften von Parfüms oder zu parfümierenden Produkten.

**Patentansprüche für folgenden Vertragsstaat : ES**

**1.** Riechstoffkompositionen, enthaltend ungesättigte Nitrile der allgemeinen Formel I

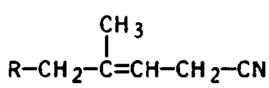

(I),

in der
R für einen verzweigten oder unverzweigten Alkylrest mit 4 bis 10 C-Atomen oder einen Phenylring steht, der durch eine Alkylgruppe mit 1 bis 4 C-Atomen, eine oder mehrere Alkoxygruppen mit 1 bis 3 C-Atomen oder eine Alkylendioxygruppe substituiert ist.

**2.** Riechstoffkompositionen, enthaltend ungesättigte Nitrile der allgemeinen Formel I gemäß Anspruch 1, in der R für

$$-(CH_2)_n-CH_3$$

steht, worin n eine ganze Zahl von 5 bis 8 bedeutet.

**3.** Riechstoffkompositionen, enthaltend ungesättigte Nitrile der allgemeinen Formel I gemäß Anspruch 1, in der R einen aromatischen Rest der Struktur

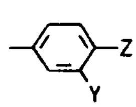

bedeutet, worin Y = H, wenn Z für $-CH_3$;

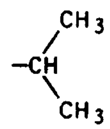 ; 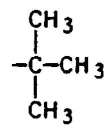 oder $-O-CH_3$

steht oder Y und Z für $-O-CH_3$ stehen oder aber Y und Z zusammen für den Rest

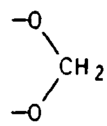

stehen.

4. Riechstoffkompositionen, enthaltend 4,7,9,9-Tetramethyl-dec-3-en-nitril.

5. Riechstoffkompositionen, enthaltend 4-Methyl-tridec-3-en-nitril.

6. Riechstoffkompositionen, enthaltend 4-Methyl-dodec-3-en-nitril.

7. Riechstoffkompositionen, enthaltend 5-(4-Methyl-phenyl)-4-methyl-pent-3-en-nitril.

8. Riechstoffkompositionen, enthaltend 5-(4-tert.-Butyl-phenyl)-4-methyl-pent-3-en-nitril.

9. Verfahren zur Herstellung der ungesättigten Nitrile der allgemeinen Formel I gemäß Anspruch 1, dadurch gekennzeichnet, daß man Aldehyde der allgemeinen Formel II

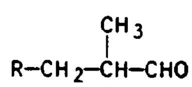 (II),

in der R die in Anspruch 1 angegebene Bedeutung hat, in einer Knoevenagel-Reaktion mit Cyanessigsäure umsetzt.

10. Verfahren zur Herstellung der ungesättigten Nitrile der allgemeinen Formel I gemäß Anspruch 1, dadurch gekennzeichnet, daß man Allylhalogenide der allgemeinen Formel III

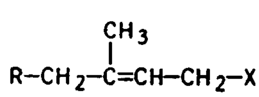 (III),

in der R die in Anspruch 1 angegebene Bedeutung hat und X für Cl, Br oder J steht, ggf. unter Phasentransferkatalyse mit Cyanidionen umsetzt.

11. Verwendung der ungesättigten Nitrile der allgemeinen Formel I gemäß Anspruch 1 zur Verleihung, Verbesserung oder Modifizierung der Dufteigenschaften von Parfüms oder zu parfümierenden Produkten.

## Claims

**Claims for the following Contracting States : CH, DE, FR, GB, LI, NL**

1. An unsaturated nitrile of the formula I

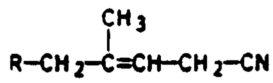 (I)

where R is a branched or straight-chain alkyl radical of 4 to 10 carbon atoms or a phenyl ring which is substituted by alkyl of 1 to 4 carbon atoms, one or more alkoxy groups of 1 to 3 carbon atoms or alkylenedioxy.

2. An unsaturated nitrile of the formula I as claimed in claim 1, where R is

$$-(CH_2)_n-CH_3$$

and n is an integer of from 5 to 8.

3. An unsaturated nitrile of the formula I as claimed in claim 1, where R is an aromatic radical of the structure

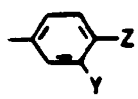

in which Y is H if Z is $-CH_3$;

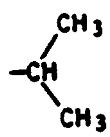 ; 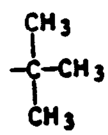 or $-O-CH_3$

or Y and Z are each $-O-CH_3$ or Y and Z together form the radical

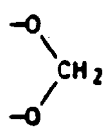

4. 4,7,9,9-Tetramethyldec-3-enenitrile.

5. 4-Methyltridec-3-enenitrile.

6. 4-Methyldodec-3-enenitrile.

7. 5-(4-Methylphenyl)-4-methylpent-3-enenitrile.

8. 5-(4-Tert-butylphenyl)-4-methylpent-3-enenitrile.

9. A process for the preparation of an unsaturated nitrile of the formula I as claimed in claim 1, wherein an aldehyde of the formula II

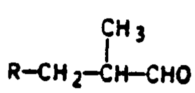 (II)

where R has the meanings stated in claim 1, is subjected to a Knoevenagel reaction with cyanoacetic acid.

10. A process for the preparation of an unsaturated nitrile of the formula I as claimed in claim 1, wherein an allyl halide of the formula III

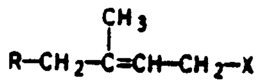

(III)

where R has the meanings stated in claim 1 and X is Cl, Br or I, is reacted with cyanide ions, if necessary under phase-transfer catalysis.

11. Use of an unsaturated nitrile of the formula I as claimed in claim 1 for imparting fragrance properties to perfumes or to products to be perfumed, or for improving or modifying the fragrance properties of perfumes or of said products.

**Claims for the following Contracting State : ES**

1. A scent composition containing unsaturated nitriles of the formula I

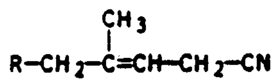

(I)

where R is a branched or straight-chain alkyl radical of 4 to 10 carbon atoms or a phenyl ring which is substituted by alkyl of 1 to 4 carbon atoms, one or more alkoxy groups of 1 to 3 carbon atoms or alkylenedioxy.

2. A scent composition comprising unsaturated nitriles of the formula I as claimed in claim 1, where R is

$$-(CH_2)_n-CH_3$$

and n is an integer of from 5 to 8.

3. A scent composition comprising unsaturated nitriles of the formula I as claimed in claim 1, where R is an aromatic radical of the structure

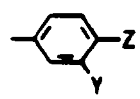

in which Y is H is Z if $-CH_3$;

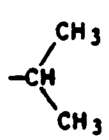

;

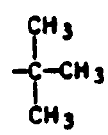

or $-O-CH_3$

or Y and Z are each $-O-CH_3$ or Y and Z together form the radical

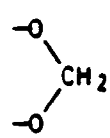

4. A scent composition comprising 4,7,9,9-tetramethyldec-3-enenitrile.

5. A scent composition comprising 4-methyltridec-3-enenitrile.

6. A scent composition comprising 4-methyldodec-3-enenitrile.

7. A scent composition comprising 5-(4-methylphenyl)-4-methylpent-3-enenitrile.

8. A scent composition comprising 5-(4-tert-butylphenyl)-4-methylpent-3-enenitrile.

9. A process for the preparation of an unsaturated nitrile of the formula I as claimed in claim 1, wherein an aldehyde of the formula II

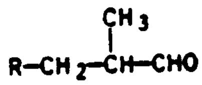 (II)

where R has the meanings stated in claim 1, is subjected to a Knoevenagel reaction with cyanoacetic acid.

10. A process for the preparation of an unsaturated nitrile of the formula I as claimed in claim 1, wherein an allyl halide of the formula III

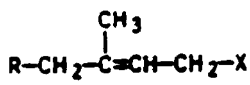 (III)

where R has the meanings stated in claim 1 and X is Cl, Br or I, is reacted with cyanide ions, if necessary under phase-transfer catalysis,

11. Use of an unsaturated nitrile of the formula I as claimed in claim 1 for imparting fragrance properties to perfumes or to products to be perfumed, or for improving or modifying the fragrance properties of perfumes or of said products.

## Revendications

### Revendications pour les Etats contractants suivants : CH, DE, FR, GB, LI, NL

1. Nitriles insaturés de formule générale I

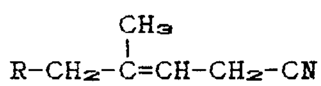 (I),

dans laquelle

R est mis pour un reste alkyle ramifié ou non ramifié avec de 4 à 10 atomes de carbone ou pour un noyau phényle qui est substitué par un groupement alkyle avec de 1 à 4 atomes de carbone, un ou plusieurs groupements alcoxy avec de 1 à 3 atomes de carbone ou un groupement alkylènedioxy.

2. Nitriles insaturés de formule générale I selon la revendication 1, dans lesquels R est mis pour

$$-(CH_2)_n-CH_3$$

où n représente un entier de 5 à 8.

3. Nitriles insaturés de formule générale I selon la revendication 1, dans lesquels R représente un reste aromatique de formule

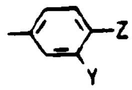

où Y est H quand Z est mis pour $CH_3$;

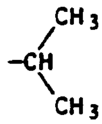 ; 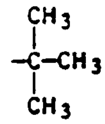 ou $-O-CH_3$

ou bien Y et Z sont mis pour $-O-CH_3$ ou bien encore Y et Z sont mis ensemble pour le reste

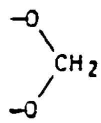

4. 4,7,9,9-Tétraméthyldéc-3-ènenitrile.

5. 4-Méthyltridéc-3-ènenitrile.

6. 4-Méthyldodéc-3-ènenitrile.

7. 5-(4-Méthylphényl)-4-méthylpent-3-ènenitrile.

8. 5-(4-tert.-Butylphényl)-4-méthylpent-3-ènenitrile.

9. Procédé de préparation des nitriles insaturés de formule générale I selon la revendication 1, caractérisé en ce qu'on fait réagir avec de l'acide cyanoacétique dans une réaction de Knoevenagel des aldéhydes de formule générale II

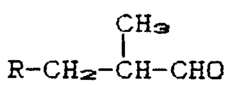 (II),

dans laquelle R a la signification donnée dans la revendication 1.

10. Procédé de préparation des nitriles insturés de formule générale I selon la revendication 1, caractérisé en ce qu'on fait réagir avec des ions cyanure, éventuellement sous catalyse de transfert de phase, des halogénures d'allyle de formule générale III

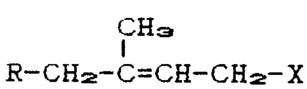 (III),

dans laquelle R a la signification donnée dans la revendication 1 et X est mis pour Cl, Br ou I.

11. Utilisation des nitriles insaturés de formule générale I selon la revendication 1 pour donner des propriétés odorantes à des parfums, les améliorer ou les modifier ou pour parfumer des produits.

**Revendications pour l'Etat contractant suivant : ES**

1. Compositions odorantes, contenant des nitriles insaturés de formule générale I

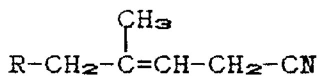 (I),

dans laquelle
R est mis pour un reste alkyle ramifié ou non ramifié avec de 4 à 10 atomes de carbone ou pour un noyau phényle qui est substitué par un groupement alkyle avec de 1 à 4 atomes de carbone, un ou plusieurs groupements alcoxy avec de 1 à 3 atomes de carbone ou un groupement alkylènedioxy.

2. Compositions odorantes contenant des nitriles insaturés de formule générale I selon la revendication 1, formule dans laquelle R est mis pour

$$-(CH_2)_n-CH_3$$

où n représente un entier de 5 à 8.

3. Compositions odorantes contenant des nitriles insaturés de formule générale I selon la revendication 1, formule dans laquelle R représente un reste aromatique de formule

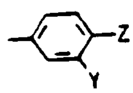

où Y est H quand Z est mis pour $CH_3$;

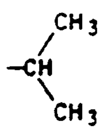 ; 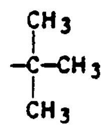 ou $-O-CH_3$

ou bien Y et Z sont mis pour $-O-CH_3$ ou bien encore Y et Z sont mis ensemble pour le reste

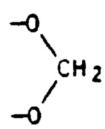

4. Compositions odorantes contenant du 4,7,9,9-tétraméthyldéc-3-ènenitrile.

5. Compositions odorantes contenant du 4-méthyltridéc-3-ènenitrile.

6. Compositions odorantes contenant du 4-méthyldodéc-3-ènenitrile.

7. Compositions odorantes contenant du 5-(4-méthylphényl)-4-méthylpent-3-ènenitrile.

8. Compositions odorantes contenant du 5-(4-tert.-butylphényl)-4-méthylpent-3-ènenitrile.

9. Procédé de préparation des nitriles insaturés de formule générale I selon la revendication 1, caractérisé en ce qu'on fait réagir avec de l'acide cyanoacétique dans une réaction de Knoevenagel des aldéhydes de formule générale II

$$\begin{array}{l} \qquad\qquad\;\; CH_3 \\ R-CH_2-CH-CHO \end{array} \qquad (II),$$

dans laquelle R a la signification donnée dans la revendication 1.

**10.** Procédé de préparation des nitriles insaturés de formule générale I selon la revendication 1, caractérisé en ce qu'on fait réagir avec des ions cyanure, éventuellement sous catalyse de transfert de phase, des halogénures d'allyle de formule générale III

$$\begin{array}{l} \qquad\qquad\;\; CH_3 \\ R-CH_2-C=CH-CH_2-X \end{array} \qquad (III),$$

dans laquelle R a la signification donnée dans la revendication 1 et X est mis pour Cl, Br, I.

**11.** Utilisation des nitriles insaturés de formule générale I selon la revendication 1 pour donner des propriétés odorantes à des parfum, les améliorer ou les modifier ou pour parfumer des produits.